(19) Europäisches Patentamt
European Patent Office
Office européen des brevets

(11) Veröffentlichungsnummer: **0 673 919 A1**

# (12) EUROPÄISCHE PATENTANMELDUNG

(21) Anmeldenummer: **95103934.6**

(22) Anmeldetag: **17.03.95**

(51) Int. Cl.[6]: **C07C 213/02**, C07D 295/096

(30) Priorität: **26.03.94 DE 4410660**

(43) Veröffentlichungstag der Anmeldung:
**27.09.95 Patentblatt 95/39**

(84) Benannte Vertragsstaaten:
**CH DE ES FR GB IT LI NL**

(71) Anmelder: **BASF AKTIENGESELLSCHAFT**
**D-67056 Ludwigshafen (DE)**

(72) Erfinder: **Reinhardt, Robert, Dr.**
**Freiheitsstrasse 12**
**D-67149 Meckenheim (DE)**
Erfinder: **Reichelt, Helmut, Dr.**
**Johann-Gottlieb-Fichte-Strasse 56**
**D-67435 Neustadt (DE)**
Erfinder: **Merger, Roland, Dr.**
**Blumenstrasse 15**
**D-76669 Bad Schönborn (DE)**

## (54) Verfahren zur Herstellung von carbocyclischen m-Amino-hydroxyaromaten.

(57) Verfahren zur Herstellung von carbocyclischen m-Amino-hydroxyaromaten durch Umsetzung der entsprechenden o- oder m-Halogen-hydroxyaromaten oder deren Metallsalzen mit primären oder sekundären Aminen in Gegenwart einer Base und gegebenenfalls eines Verdünnungsmittels.

EP 0 673 919 A1

Die vorliegende Erfindung betrifft ein neues Verfahren zur Herstellung von carbocyclischen m-Amino-hydroxyaromaten durch Umsetzung der entsprechenden o- oder m-Halogen-hydroxyaromaten mit primären oder sekundären Aminen.

Aus J. Amer. Chem. Soc., Band 74, Seiten 3027 bis 3029, 1952, ist bekannt, daß bei der Behandlung von o-Bromphenol mit der doppeltmolaren Menge an Lithiumdiethylamid in siedendem Diethylether m-Diethylaminophenol entsteht. Das dort beschriebene Verfahren ist aber für eine technische Synthese völlig unbrauchbar, da m-Diethylaminophenol nur in einer Ausbeute von 15 % gebildet wird.

Aufgabe der vorliegenden Erfindung war es, ein neues Verfahren zur Herstellung von carbocyclischen m-Amino-hydroxyaromaten bereitzustellen, das die Zielprodukte auf einfache Weise und in hoher Ausbeute und Reinheit liefert.

Es wurde nun gefunden, daß die Herstellung von carbocyclischen m-Amino-hydroxyaromaten vorteilhaft gelingt, wenn man carbocyclische o- oder m-Halogen-hydroxyaromaten oder deren Metallsalze mit primären oder sekundären Aminen in Gegenwart einer Base und gegebenenfalls eines Verdünnungsmittels umsetzt.

Carbocyclische o- oder m-Halogen-hydroxyaromaten sind z.B. o- oder m-Halogenphenole oder o- oder m-Halogen-hydroxynaphthaline.

Primäre oder sekundäre Amine sind z.B. primäre oder sekundäre gesättigte oder ungesättigte aliphatische, cycloaliphatische oder aromatische Amine.

Die m-Aminophenole oder m-Amino-hydroxynaphthaline gehorchen vorzugsweise der Formel I

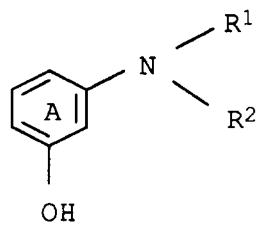

(I),

in der

$R^1$ und $R^2$ unabhängig voneinander jeweils $C_1$-$C_{18}$-Alkyl, das gegebenenfalls substituiert ist und durch 1 bis 3 Sauerstoffatome in Etherfunktion oder 1 bis 3 $C_1$-$C_4$-Alkyliminogruppen unterbrochen sein kann, $C_3$-$C_7$-Cycloalkyl, $C_3$-$C_{18}$-Alkenyl, gegebenenfalls substituiertes Phenyl oder $R^1$ und $R^2$ zusammen mit dem sie verbindenden Stickstoffatom einen 5- oder 6-gliedrigen gesättigten heterocyclischen Rest, der ein weiteres Heteroatom aufweisen kann, oder $R^1$ auch Wasserstoff oder $C_1$-$C_8$-Alkanoyl bedeuten und

der Ring A gegebenenfalls substituiert ist und benzoanelliert sein kann.

Die o- oder m-Halogenphenole oder o- oder m-Halogen-hydroxynaphthaline gehorchen vorzugsweise der Formel II

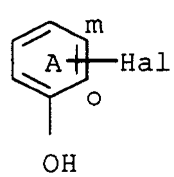

(II),

in der Hal Halogen bedeutet und der Ring A die obengenannte Bedeutung besitzt (in freier Form oder als Metallsalz), wobei die o-Verbindungen von besonderer Bedeutung sind.

Die primären oder sekundären aliphatischen, cycloaliphatischen oder aromatischen Amine gehorchen vorzugsweise der Formel III

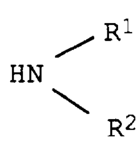

(III),

in der $R^1$ und $R^2$ jeweils die obengenannte Bedeutung besitzen.

Alle in den obengenannten Formeln auftretenden Alkyl- und Alkenylgruppen können sowohl geradkettig als auch verzweigt sein.

Im erfindungsgemäßen Sinn sind unter Alkenylresten im wesentlichen solche Reste zu verstehen, die 1 bis 3 Doppelbindungen aufweisen.

Wenn in den obengenannten Formeln substituierte Alkylgruppen auftreten, so können als Substituenten z.B. Hydroxy, Amino oder Phenyl, in Betracht kommen. Die Alkylgruppen weisen dabei in der Regel 1 oder 2 Substituenten auf.

Wenn in den obengenannten Formeln substituierte Phenylgruppen auftreten oder wenn der Ring A substituiert ist, so können als Substituenten z.B. $C_1$-$C_4$-Alkyl, Hydroxy, Halogen oder $C_1$-$C_4$-Alkoxy in Betracht kommen. Die Phenylgruppen oder Ring A weisen dabei in der Regel 1 bis 3 Substituenten auf.

Wenn $R^1$ und $R^2$ zusammen mit dem verbindenden Stickstoffatom einen 5- oder 6-gliedrigen gesättigten heterocyclischen Rest, der ein weiteres Heteroatom aufweisen kann, bedeuten, so können dafür z.B. Pyrrolidinyl, Piperidinyl, Morpholinyl, Piperazinyl oder N-($C_1$-$C_4$-Alkyl)piperazinyl in Betracht kommen.

Reste $R^1$ und $R^2$ sind z.B. Methyl, Ethyl, Propyl, Isopropyl, Butyl, Isobutyl, sec-Butyl, Pentyl, Isopentyl, Neopentyl, tert-Pentyl, Hexyl, 2-Methylpentyl, Heptyl, Octyl, 2-Ethylhexyl, Isooctyl, Nonyl, Isononyl, Decyl, Isodecyl, Undecyl, Dodecyl, Tridecyl, 3,5,5,7-Tetramethylnonyl, Isotridecyl (die obigen Bezeichnungen Isooctyl, Isononyl, Isodecyl und Isotridecyl sind Trivialbezeichnungen und stammen von den nach der Oxosynthese erhaltenen Alkoholen - vgl. dazu Ullmann's Encyclopedia of Industrial Chemistry, 5th Edition, Vol. Al, Seiten 290 bis 293, sowie Vol. A 10, Seiten 284 und 285), Tetradecyl, Pentadecyl, Hexadecyl, Heptadecyl, Octadecyl, 2-Methoxyethyl, 2-Ethoxyethyl, 2-Propoxyethyl, 2-Isopropoxyethyl, 2-Butoxyethyl, 2- oder 3-Methoxypropyl, 2- oder 3-Ethoxypropyl, 2- oder 3-Propoxypropyl, 2- oder 3-Butoxypropyl, 2- oder 4-Methoxybutyl, 2- oder 4-Ethoxybutyl, 2- oder 4-Propoxybutyl, 2- oder 4-Butoxybutyl, 2-Hydroxyethyl, 2- oder 3-Hydroxypropyl, 2- oder 4-Hydroxybutyl, 5-Hydroxypentyl, 6-Hydroxyhexyl, 5-Hydroxy-3-oxapentyl, Benzyl, 1-Phenylethyl, 2-Phenylethyl, 2-Aminoethyl, 2- oder 3-Aminopropyl, 2- oder 4-Aminobutyl, Cyclopropyl, Cyclobutyl, Cyclopentyl, Cyclohexyl, Methylcyclohexyl, Cycloheptyl, Allyl, Prop-1-en-1-yl, Methallyl, Ethallyl, Pentenyl, Pentadienyl, Hexadienyl, 3,7-Dimethylocta-1,6-dien-1-yl, Undec-10-en-1-yl, 6,10-Dimethylundeca-5,9-dien-2-yl, 3,7,11-Trimethyldodeca-1,6,10-trien-1-yl, 3,7,11-Trimethyldodeca-2,6, 10-trien-1-yl, Octadec-9-en-1-yl, Octadeca-9,12-dien-1-yl, Octadeca-9,12,15-trien-1-yl, 6,10,14-Trimethylpentadeca-5,9, 13-trien-2-yl, Phenyl, 2-, 3- oder 4-Methylphenyl, 2-, 3- oder 4-Ethylphenyl, 2,4-Dimethylphenyl, 2-, 3- oder 4-Methoxyphenyl, 2-, 3- oder 4-Ethoxyphenyl, 2,4-Dimethoxyphenyl, 2-, 3- oder 4-Chlorphenyl, 2,4-Dichlorphenyl, 3,6-Dioxaheptyl, 3,6-Dioxaoctyl, 4,8-Dioxanonyl, 3,7-Dioxaoctyl, 3,7-Dioxanonyl, 4,7-Dioxaoctyl, 4,7-Dioxanonyl, 4,8-Dioxadecyl, 3,6,8-Trioxadecyl, 3,6,9-Trioxaundecyl, 2-Dimethylaminoethyl, 2-Diethylaminoethyl, 2- oder 3-Dimethylaminopropyl, 2- oder 3-Diethylaminopropyl, 2- oder 4-Dimethylaminobutyl, 2- oder 4-Diethylaminobutyl, 3,6-Dimethyl-3,6-diazaheptyl, 3,6,9-Trimethyl-3,6,9-triazadecyl, 2-(1-Methoxyethoxy)-ethyl, 2-(1-Ethoxyethoxy)ethyl, 2-(1-Isobutoxyethoxy)ethyl, 2- oder 3-(1-Methoxyethoxy)propyl, 2- oder 3-(1-Ethoxyethoxy)propyl oder 2- oder 3-(1-Isobutoxyethoxy)propyl.

Reste $R^1$ sind weiterhin z.B. Formyl, Acetyl, Propionyl, Butyryl, Isobutyryl, Pentanoyl, Hexanoyl, Heptanoyl, Octanoyl oder 2-Ethylhexanoyl.

Carbocyclische o- oder m-Halogen-hydroxyaromaten sind z.B. die entsprechenden Fluor-, Chlor- oder Bromverbindungen. Die Verwendung der Chlor- oder Bromverbindungen ist bevorzugt, wobei die Chlorverbindungen besonders hervorzuheben sind.

Bezogen auf die Formel II bedeutet dies, daß solche Verbindungen der Formel II bevorzugt verwendet werden, in der Hal, Chlor oder Brom, insbesondere Chlor, bedeutet.

Wie oben ausgeführt, können die carbocyclischen o- oder m-Halogen-hydroxyaromaten im erfindungsgemäßen Verfahren entweder in freier Form oder in Form ihrer Metallsalze angewandt werden.

Geeignete Metallsalze sind z.B. die Alkali- oder Erdalkalisalze, wie die Lithium-, Natrium-, Kalium-, Magnesium- oder Calciumsalze.

Wenn die carbocyclischen o- oder m-Halogen-hydroxyaromaten in Form ihrer Metallsalze angewandt werden, so ist die Verwendung der Alkalisalze, insbesondere der Natrium- oder Kaliumsalze, hervorzuheben.

Geeignete Basen, die im erfindungsgemäßen Verfahren zur Anwendung kommen können, sind z.B. Alkali- oder Erdalkaliamide, Alkali- oder Erdalkalialkoholate, Alkali- oder Erdalkalihydride oder alkali- oder erdalkalimetallorganische Verbindungen. Die Verwendung von Alkali- oder Erdalkalihydroxiden oder Alkali- oder Erdalkalioxiden ist ebenfalls möglich.

Geeignete Alkali- oder Erdalkalisalze sind z.B. die entsprechenden Lithium-, Natrium-, Kalium-, Magnesium- oder Calciumsalze. Die Verwendung der Alkalisalze, insbesondere der Natrium- oder Kaliumsalze ist bevorzugt.

Als alkali- oder erdalkalimetallorganische Verbindungen sind insbesondere die lithiumorganischen Verbindungen, z.B. Methyl-, Ethyl-, Propyl-, Butyl- oder Phenyllithium, zu nennen.

Geeignete Amide sind z.B. die unsubstituierten Alkali- oder Erdalkaliamide oder diejenigen Alkali- oder Erdalkaliamide, die sich von Aminen der Formel $NHR^1R^2$ ableiten, worin $R^1$ und $R^2$ jeweils die obengenannte Bedeutung besitzen.

Geeignete Alkoholate sind die Alkali- oder Erdalkalialkoholate, die sich von $C_1$-$C_4$-Alkanolen, wie Methanol, Ethanol, Propanol, Isopropanol, Butanol, Isobutanol, sec-Butanol oder tert-Butanol, ableiten.

Geeignete Verdünnungsmittel, die im erfindungsgemäßes Verfahren zur Anwendung gelangen können, sind z.B. die primären oder sekundären Amine, die als Reaktionspartner auftreten, in überschüssiger Form. Als Verdünnungsmittel können aber auch inerte Verdünnungsmittel, beispielsweise Ether, wie Diethylether, Methyl-tert-butylether, 1,2-Dimethoxyethan, 1,2-Diethoxyethan, Tetrahydrofuran oder Dioxan, Carbonsäureamide, wie N,N-Dimethylformamid oder N,N-Dimethylacetamid, oder aliphatische oder aromatische Kohlenwasserstoffe, wie Hexan, Heptan, Octan, Isooctan, Petrolether, Ligroin, Toluol, Xylol, Dodecylbenzol, Diisopropylnaphthalin oder ein Gemisch höherer Aromaten, das unter den Namen Shellsol® AB (Fa. Shell) handelsüblich ist, zur Anwendung gelangen.

Bevorzugt verwendet man im erfindungsgemäßen Verfahren als Base Alkaliamide, vorzugsweise Natrium- oder Kaliumamid, wobei Natriumamid besonders hervorzuheben ist.

Als bevorzugte Verdünnungsmittel im erfindungsgemäßen Verfahren dienen die zur Reaktion gelangenden primären oder sekundären Amine in überschüssiger Form.

Eine bevorzugte Ausführungsform besteht darin, daß man Amine der Formel III zur Reaktion bringt, in der $R^1$ und $R^2$ unabhängig voneinander jeweils $C_1$-$C_{15}$-Alkyl, das gegebenenfalls substituiert ist und durch 1 bis 3 Sauerstoffatome in Etherfunktion unterbrochen sein kann, Cyclohexyl, gegebenenfalls substituiertes Phenyl oder $R^1$ und $R^2$ zusammen mit dem sie verbindenden Stickstoffatom einen 5- oder 6-gliedrigen gesättigten heterocyclischen Rest, der ein weiteres Heteroatom aufweisen kann, oder $R^1$ auch Wasserstoff bedeuten.

Eine bevorzugte Ausführungsform besteht weiterhin darin, daß man o-Halogenphenole der Formel IIa

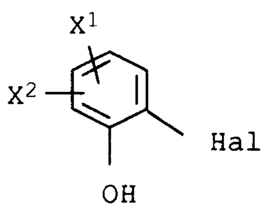

(IIa)

zur Reaktion bringt, in der

Hal Chlor oder Brom, insbesondere Chlor, und $X^1$ und $X^2$ unabhängig voneinander jeweils Wasserstoff oder $C_1$-$C_4$-Alkyl, insbesondere Wasserstoff, bedeuten.

Besonders bevorzugt verwendet man im erfindungsgemäßen Verfahren Amine der Formel III, in der $R^1$ und $R^2$ unabhängig voneinander jeweils $C_1$-$C_6$-Alkyl, Cyclohexyl, Phenyl oder $R^1$ und $R^2$ zusammen mit dem sie verbindenden Stickstoffatom einen 5- oder 6-gliedrigen gesättigten heterocyclischen Rest, der ein weiteres Heteroatom aufweisen kann oder $R^1$ auch Wasserstoff bedeuten.

Insbesondere hervorzuheben ist die Verwendung von Aminen der Formel III, in der $R^1$ und $R^2$ unabhängig voneinander jeweils $C_1$-$C_6$-Alkyl, insbesondere $C_1$-$C_4$-Alkyl, oder $R^1$ und $R^2$ zusammen mit dem sie verbindenden Stickstoffatom Pyrrolidinyl, Piperidinyl oder Morpholinyl oder $R^1$ auch Wasserstoff bedeuten.

Bezogen auf 1 mol carbocyclischen o- oder m-Halogen-hydroxyaromaten (in freier Form) können im neuen Verfahren 2 bis 20 mol, vorzugsweise 2 bis 10 mol und insbesondere 2,5 bis 5 mol Base zur Anwendung gelangen.

Bezogen auf 1 mol carbocyclischen o-Halogen-hydroxyaromaten (als Metallsalz) können im neuen Verfahren 1 bis 20 mol, vorzugsweise 1 bis 10 mol und insbesondere 1,5 bis 5 mol Base zur Anwendung gelangen.

Die primären oder sekundären Amine und die carbocyclischen o- oder m-Halogen-hydroxyaromaten sollen im neuen Verfahren mindestens im Molverhältnis 1 : 1, vorzugsweise mindestens 5 : 1 und insbesondere mindestens 15 : 1 zur Anwendung gelangen.

Da, wie oben bereits ausgeführt, die primären oder sekundären Amine gleichzeitig auch als Verdünnungsmittel dienen können, gibt es im Prinzip keine feste Obergrenze bezüglich der Menge ihrer

Anwendung, jedoch sollte aus technischen Gründen im allgemeinen das Molverhältnis Amin : o- oder m-Halogen-hydroxyaromat nicht höher als 100:1 sein.

Wie oben weiterhin ausgeführt, können jedoch auch inerte Verdünnungsmittel zur Anwendung gelangen. Bezogen auf das Gewicht der carbocyclischen o- oder m-Halogen-hydroxyaromaten wendet man im allgemeinen 0 bis 2000 Gew.-% an inertem Verdünnungsmittel an.

Das erfindungsgemäße Verfahren kann bei einer Temperatur von -50 bis +300 °C, vorzugsweise 0 bis +300 °C durchgeführt werden, wobei sich die besonders bevorzugte Obergrenze der Temperatur am Siedepunkt der jeweils niedrigst siedenden Komponente im Reaktionsgemisch orientiert.

Im allgemeinen wird das erfindungsgemäße Verfahren bei atmosphärischem Druck vorgenommen, jedoch kann man auch unter leichtem Druck (bis zu ca. 20 bar) arbeiten.

Das neue Verfahren kann sowohl in kontinuierlicher als auch diskontinuierlicher Arbeitsweise durchgeführt werden.

Es wird zweckmäßig so vorgenommen, daß man, gegebenenfalls unter Schutzgasatmosphäre, z.B. Stickstoffatmosphäre, die Reaktionspartner, Base und gegebenenfalls inertes Verdünnungsmittel in beliebiger Reihenfolge in einer geeigneten Apparatur, z.B. in einer Rührapparatur, vorlegt und bei der obengenannten Temperatur bis zum gewünschten Umsetzungsgrad des o- oder m-Halogen-hydroxyaromaten behandelt.

Danach wird auf Raumtemperatur abgekühlt, mit Wasser oder Methanol versetzt und das Reaktionsgemisch nach an sich bekannten Methoden, wie auch in den Beispielen näher beschrieben, aufgearbeitet, beispielsweise durch Destillation, Kristallisation, Extraktion oder chromatographische Methoden.

Das erfindungsgemäße Verfahren ist technisch einfach durchzuführen und liefert die Zielprodukte in guter Ausbeute und Isomerenreinheit. Im Vergleich zu den bekannten technischen Methoden für die Herstellung von m-Aminophenolen zeichnet es sich durch seine geringe Stufenanzahl (einstufig) aus.

Bei den mittels des erfindungsgemäßen Verfahrens hergestellten carbocyclischen m-Amino-hydroxyaromaten handelt es sich um wertvolle Zwischenprodukte, beispielsweise für die Herstellung von Fluoranen, die als Farbbildner zur Anwendung kommen (siehe z.B. US-A-3 873 573).

Die folgenden Beispiele sollen die Erfindung näher erläutern.

Beispiel 1

Unter Stickstoff gab man zu 150 ml trockenem Diethylamin 6,2 g (150 mmol) 95 gew.-%iges kristallines Natriumamid und rührte 5 min bei 20 °C. Dann fügte man 6,4 g (50 mmol) 2-Chlorphenol zu und erhitzte die Reaktionsmischung 3 h unter Rühren zum Rückfluß. Nach Abkühlen auf 20 °C versetzte man zur Vernichtung überschüssigen Natriumamids vorsichtig mit 50 ml Wasser und destillierte bis 100 °C Innentemperatur Diethylamin ab. Der Destillationssumpf wurde mit Wasser versetzt, mit konz. Salzsäure auf einen pH-Wert von 7,0 gestellt und mit Dichlormethan extrahiert. Destillative Entfernung des Lösungsmittels und Trocknen der vereinigten Extrakte mit Natriumsulfat ergab 7,7 g eines roten Öls, das nach GC 1,9 g (30 % d.Th.) 2-Chlorphenol und 5,4 g (65 % d.Th.) 3-Diethylaminophenol enthält. Bezogen auf den Umsatz an 2-Chlorphenol beträgt die Ausbeute an 3-Diethylaminophenol 93 %.

Beispiel 2

Unter Stickstoff gab man zu 200 ml trockenem Diethylamin 6,2 g (150 mmol) 95 gew.-%iges kristallines Natriumamid und rührte 5 min bei 20 °C. Dann fügte man 6,4 g (50 mmol) 3-Chlorphenol zu und erhitzte die Reaktionsmischung 5 h unter Rühren zum Rückfluß. Nach Abkühlen auf 20 °C versetzte man zur Vernichtung überschüssigen Natriumamids vorsichtig mit 5 ml Wasser und destillierte bis 100 °C Innentemperatur Diethylamin ab. Der Destillationssumpf wurde mit konz. Salzsäure auf einen pH-Wert von 7,5 gestellt und nach Zugabe von 5 ml Wasser und 150 g Dichlormethan klärfiltriert. Nach Abtrennung der organischen Phase destillierte man aus dieser Dichlormethan und weitere flüchtige Bestandteile zunächst unter Normaldruck, dann unter vermindertem Druck ab. Man erhielt 14,0 g dunkles Öl, das nach GC 1,6 g (25 % d.Th.) 3-Chlorphenol und 5,9 g (72 % d. Th.) 3-Diethylaminophenol enthält. Bezogen auf den Umsatz an 3-Chlorphenol beträgt die Ausbeute an 3-Diethylaminophenol 96 %.

Beispiel 3

Unter Stickstoff gab man zu 150 ml trockenem Di-n-butylamin 6,2 g (150 mmol) 95 gew.-%iges kristallines Natriumamid und rührte 5 min bei 20 °C. Dann fügte man 6,4 g (50 mmol)2-Chlorphenol zu, rührte die Reaktionsmischung 10 h bei 80 °C und 4 h bei 100 °C. Nach Abkühlen auf 20 °C versetzte man

zur Vernichtung überschüssigen Natriumamids vorsichtig mit Wasser und extrahierte mit Diethylether. Aus den vereinigten Extrakten destillierte man Diethylether und unter vermindertem Druck Di-n-butylamin weitgehend ab. Man erhielt 10,9 g eines Öls, das aus 3,1 g Di-n-butylamin und 7,8 g (70 % d.Th.) 3-(Di-n-butylamino)phenol besteht (NMR).

Beispiel 4

Unter Stickstoff gab man zu 150 ml trockenem Morpholin 6,2 g (150 mmol) 95 gew.-%iges kristallines Natriumamid und rührte 5 min bei 20 °C. Dann fügte man 6,4 g (50 mmol) 2-Chlorphenol zu und erhitzte die Reaktionsmischung 2 h auf 80 °C. Nach Abkühlen auf 20 °C versetzte man zur Vernichtung überschüssigen Natriumamids vorsichtig mit 100 ml Wasser und destillierte unter vermindertem Druck Morpholin ab. Die Lösung des festen Rückstands in Wasser wurde mit konz. Salzsäure auf einen pH-Wert von 7,0 gestellt und mit Dichlormethan extrahiert. Destillative Entfernung des Lösungsmittels nach Trocknen der vereinigten Extrakte mit Natriumsulfat ergab 7,2 g hellbraune Kristalle mit Schmp. 120 bis 123 °C, die nach GC 6,1 g (68 % d.Th.) 3-Morpholinophenol und 0,6 g Morpholin enthalten.

Beispiel 5

Unter Stickstoff gab man zu 125 ml trockenem n-Butylamin 3,1 g (75 mmol) 95 gew.-%iges kristallines Natriumamid und rührte 5 min bei 20 °C. Dann fügte man 3,6 g (25 mmol) 2-Chlor-4-methylphenol zu und erhitzte die Reaktionsmischung 2 h unter Rühren zum Rückfluß. Nach Abkühlen auf 20 °C versetzte man zur Vernichtung überschüssigen Natriumamids mit 10 ml Methanol und destillierte n-Butylamin ab. Der feste Rückstand wurde mit Wasser versetzt, mit konz. Salzsäure auf einen pH-Wert von 7,0 gestellt und mit Dichlormethan extrahiert. Nach dem Trocknen der vereinigten Extrakte mit Natriumsulfat destillierte man das Lösungsmittel ab. Man erhielt 4,6 g eines Öls, das nach GC 0,2 g (4 % d.Th.) 2-Chlor-4-methylphenol und 3,0 g (68 % d.Th.) 3-(n-Butylamino)phenol enthält. Bezogen auf den Umsatz an 2-Chlor-4-methylphenol beträgt die Ausbeute an 3-(n-Butylamino)phenol 71 %.

Beispiel 6

32,1 g (0,25 mol) o-Chlorphenol wurden unter Stickstoff langsam zu einer Mischung von 20,5 g (0,5 mol) 95 gew.-%igem kristallinem Natriumamid und 750 ml Diethylamin getropft. Das Reaktionsgemisch wurde unter Stickstoffatmosphäre 72 h unter Rückfluß gerührt. Die Aufarbeitung erfolgte durch Hydrolyse mit 100 ml gesättigter wäßriger Ammoniumchloridlösung, Abdestillieren des überschüssigen Diethylamins, Neutralisation mit verdünnter Salzsäure bis zu einem pH-Wert von 7,5, Extraktion des Rückstandes mit insgesamt 600 ml Methylenchlorid, Trocknen über Natriumsulfat und Einengen der organischen Phase. Danach wurde das so erhaltene rohe Öl (46,3 g) destilliert, wobei 31,5 g (76 %) 3-Diethylaminophenol (Gehalt >96 % (GC)) erhalten wurden.

In analoger Weise können die in der folgenden Tabelle aufgeführten Verbindungen erhalten werden.

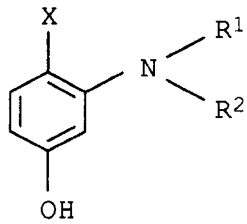

| Bsp. Nr. | X | $NR^1R^2$ |
| --- | --- | --- |
| 7 | $CH_3$ | $NH-C_2H_5$ |
| 8 | $CH_3$ | $NH-C_3H_7$ |
| 9 | $CH_3$ | $NH-C_5H_{11}$ |
| 10 | $CH_3$ | $NH-C_6H_{13}$ |
| 11 | $CH_3$ | 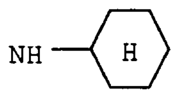 |
| 12 | $CH_3$ | $NH-C_6H_5$ |
| 13 | H | Pyrrolidino |
| 14 | H | Piperidino |
| 15 | H | 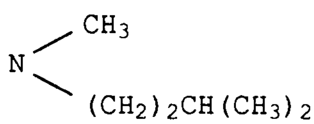 |
| 16 | H | 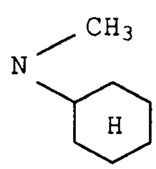 |

## Patentansprüche

1. Verfahren zur Herstellung von carbocyclischen m-Aminohydroxyaromaten, dadurch gekennzeichnet, daß man carbocyclische o- oder m-Halogen-hydroxyaromaten oder deren Metallsalze mit primären oder sekundären Aminen in Gegenwart einer Base und gegebenenfalls eines Verdünnungsmittels umsetzt.

2. Verfahren nach Anspruch 1, dadurch gekennzeichnet, daß die carbocyclischen m-Amino-hydroxyaromaten der Formel I

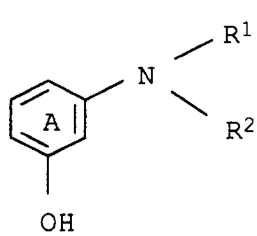 (I)

gehorchen, in der

$R^1$ und $R^2$ unabhängig voneinander jeweils $C_1$-$C_{18}$-Alkyl, das gegebenenfalls substituiert ist und durch 1 bis 3 Sauerstoffatome in Etherfunktion oder 1 bis 3 $C_1$-$C_4$-Alkyliminogruppen unterbrochen sein kann, $C_3$-$C_7$-Cycloalkyl, $C_3$-$C_{18}$-Alkenyl, gegebenenfalls substituiertes Phenyl oder $R^1$ und $R^2$ zusammen mit dem sie verbindenden Stickstoffatom einen 5- oder 6-gliedrigen gesättigten heterocyclischen Rest, der ein weiteres Heteroatom aufweisen kann, oder $R^1$ auch Wasserstoff oder $C_1$-$C_8$-Alkanoyl bedeuten und

der Ring A gegebenenfalls substituiert ist und benzoanelliert sein kann.

**3.** Verfahren nach den Ansprüchen 1 und 2, dadurch gekennzeichnet, daß man carbocyclische o- oder m-Halogenhydroxyaromaten der Formel II

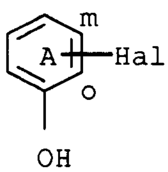 (II),

in der Hal Halogen bedeutet und der Ring A gegebenenfalls substituiert ist und benzoanelliert sein kann, oder deren Metallsalze umsetzt.

**4.** Verfahren nach den Ansprüchen 1 bis 3, dadurch gekennzeichnet, daß man primäre oder sekundären Amine der Formel III

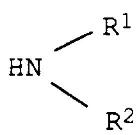 (III),

in der $R^1$ und $R^2$ jeweils die in Anspruch 2 genannte Bedeutung besitzen, zur Reaktion bringt.

**5.** Verfahren nach den Ansprüchen 1 bis 4, dadurch gekennzeichnet, daß man als Base Alkali- oder Erdalkaliamide, Alkali- oder Erdalkalialkoholate, Alkali- oder Erdalkalihydride, alkali- oder erdalkalimetallorganische Verbindungen, Alkali- oder Erdalkalihydroxide oder Alkali- oder Erdalkalioxide verwendet.

**6.** Verfahren nach den Ansprüchen 1 bis 5, dadurch gekennzeichnet, daß man die Umsetzung bei einer Temperatur von -50 bis +300 °C durchführt.

Europäisches Patentamt

# EUROPÄISCHER RECHERCHENBERICHT

Nummer der Anmeldung
EP 95 10 3934

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| A | 'Methoden der Organischen Chemie (Houben-Weyl) Band E16d' , GEORG THIEME VERLAG , STUTTGART<br>* Seite 684 - Seite 685 *<br>--- | 1 | C07C213/02<br>C07D295/096 |
| D,A | JOURNAL OF THE AMERICAN CHEMICAL SOCIETY, Bd. 74, 1952 DC US,<br>Seiten 3027-3029,<br>H. GILMAN ET AL. 'Rearrangements in Amination by Alkali Amides in Liquid Ammonia and by Lithium Dialkylamides in Ether'<br>* Seite 3028 - Seite 3029 *<br>--- | 1 | |
| A | US-A-2 062 349 (W.S. CALCOTT)<br>* Seite 2 *<br>----- | 1 | |
| | | | RECHERCHIERTE SACHGEBIETE (Int.Cl.6) |
| | | | C07C<br>C07D |

Der vorliegende Recherchenbericht wurde für alle Patentansprüche erstellt

| Recherchenort | Abschlußdatum der Recherche | Prüfer |
|---|---|---|
| DEN HAAG | 7.Juli 1995 | Pauwels, G |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)